# EUROPEAN PATENT APPLICATION

(11) **EP 2 017 337 A1**
(43) Date of publication of application: **21.01.2009**
(21) Application number: 08013946.2
(22) Date of filing: 27.04.1995
(51) Int. Cl.: C12N 15/00, C12N 15/11, C12N 15/28, C12N 15/09, C12N 15/63, C07K 14/00, C07K 14/525, C12P 21/06, C12N 5/00, C12N 5/10, C07K 14/715

(54) **Human tumor necrosis factor receptors**

(62) Divisional of application: 95917662.9
(71) Applicant: HUMAN GENOME SCIENCES, INC., Rockville, MD 20850-3338 (US)
(72) Inventor: Ni, Jian, Germantown, MD 20874 (US); Gentz, Reiner, Belo Horizonte - MG 30320450 (BR); Rosen, Craig, A., Laytonsville, MD 20882 (US)
(74) Representative: Vossius & Partner

(57) **Abstract**

A human TNF receptor polypeptide and two splice variants thereof and DNA (RNA) encoding such polypeptides and a procedure for producing such polypeptides by recombinant techniques are disclosed. Also disclosed are methods for utilizing such receptor polypeptides for screening for antagonists and agonists to the receptors and for ligands for the receptors. Also disclosed are methods for utilizing such agonists to inhibit the growth of tumors, to stimulate cellular differentiation, to mediate the immune response and anti-viral response, to regulate growth and provide resistance to certain infections. The use of the antagonists as a therapeutic to treat autoimmune diseases, inflammation, septic shock, to inhibit graft-host reactions, and to prevent apoptosis is also disclosed. Also disclosed are diagnostic assays for detecting mutations in the nucleic acid sequences and the polypeptides encoded thereby.

SEQUENCE LISTING

(1) GENERAL INFORMATION:
(i) APPLICANT: NI, ET AL.

(ii) TITLE OF INVENTION: Tumor Necrosis Factor Receptors
(iii) NUMBER OF SEQUENCES: 13

(iv) CORRESPONDENCE ADDRESS:

(A) ADDRESSEE: CARELLA, BYRNE, BAIN, GILFILLAN,
CECCHI, STEWART & OLSTEIN
(B) STREET: 6 BECKER FARM ROAD
(C) CITY: ROSELAND
(D) STATE: NEW JERSEY
(E) COUNTRY: USA
(F) ZIP: 07068

(v) COMPUTER READABLE FORM:
(A) MEDIUM TYPE: 3.5 INCH DISKETTE
(B) COMPUTER: IBM PS/2
(C) OPERATING SYSTEM: MS-DOS
(D) SOFTWARE: WORD PERFECT 5.1

(vi) CURRENT APPLICATION DATA:
(A) APPLICATION NUMBER:
(B) FILING DATE: Concurrently
(C) CLASSIFICATION:

(vii) PRIOR APPLICATION DATA
(A) APPLICATION NUMBER:
(B) FILING DATE:

(viii) ATTORNEY/AGENT INFORMATION:
(A) NAME: FERRARO, GREGORY D.
(B) REGISTRATION NUMBER: 36,134
(C) REFERENCE/DOCKET NUMBER: 325800-255

(ix) TELECOMMUNICATION INFORMATION:
(A) TELEPHONE: 201-994-1700
(B) TELEFAX: 201-994-1744

(2) INFORMATION FOR SEQ ID NO:1:

(i) SEQUENCE CHARACTERISTICS
(A) LENGTH: 881 BASE PAIRS
(B) TYPE: NUCLEIC ACID
(C) STRANDEDNESS: SINGLE
(D) TOPOLOGY: LINEAR

(ii) MOLECULE TYPE: cDNA

(xi) SEQUENCE DESCRIPTION: SEQ ID NO:1:

CCTGAGGCAT GGAGCCTCCT GGAGACTGGG GGCCTCCTCC CTGGAGATCC ACCCCCAGAA 60
CCGACGTCTT GAGGCTGGTG CTGTATCTCA CCTTCCTGGG AGCCCCCTGC TACGCCCCAG 120
CTCTGCCGTC CTGCAAGGAG GACGAGTACC CAGTGGGCTC CGAGTGCTGC CCCAAGTGCA 180
GTCCAGGTTA TCGTGTGAAG GAGGCCTGCG GGGAGCTGAC GGGCACAGTG TGTGAACCCT 240
GCCCTCCAGG CACCTACATT GCCCACCTCA ATGGCCTAAG CAAGTGTCTG CAGTGCCAAA 300
TGTGTGACCC AGCCATGGGC CTGCGCGCGA GCCGGAACTG CTCCAGGACA GAGAACGCCG 360
TGTGTGGTTG CAGCCCAGGC CACTTCTGCA TCGTCCAGGA CGGGGACCAC TGCGCCGCGT 420
GCCGCGCTTA CGCCACCTCC AGCCCGGGCC AGAGGGTGCA GAAGGGAGGC ACCGAGAGTC 480
AGGACACCCT GTGTCAGAAC TGCCCCCCGG GGACCTTCTC TCCCAATGGG ACCCTGGAGG 540
AATGTCAGCA CCAGACCAAG TGCAGCTGGC TGGTGACGAA GGCCGGAGCT GGGACCAGCA 600
GCTCCCACTG GGTATGGTGG TTTCTCTCAG GGAGCCTCGT CATCGTCATT GTTTGCTCCA 660
CAGTTGGCCT AATCATATGT GTGAAAAGAA GAAAGCCAAG GGGTGATGTA GTCAAGGTGA 720
TCGTCTCCGT CCAGCGGAAA AGACAGGAGG CAGAAGGTGA GGCCACAGTC ATTGAGGCCC 780
TGCAGGCCCC TCCGGACGTC ACCACGGTGG CCGTGGAGGA GACAATACCC TCATTCACGG 840
GGAGGAGCCC AAACCACTGA CCCACAGACT CTGCACCCCG A 881

(2) INFORMATION FOR SEQ ID NO:2:
(i) SEQUENCE CHARACTERISTICS
(A) LENGTH: 283 AMINO ACIDS
(B) TYPE: AMINO ACID
(C) STRANDEDNESS:
(D) TOPOLOGY: LINEAR

(ii) MOLECULE TYPE: PROTEIN

(xi) SEQUENCE DESCRIPTION: SEQ ID NO:2:

(2) INFORMATION FOR SEQ ID NO:3:

(i) SEQUENCE CHARACTERISTICS
(A) LENGTH: 723 BASE PAIRS
(B) TYPE: NUCLEIC ACID
(C) STRANDEDNESS: SINGLE
(D) TOPOLOGY: LINEAR

(ii) MOLECULE TYPE: Oligonucleotide

(xi) SEQUENCE DESCRIPTION: SEQ ID NO:3:

ATGGAGCCTC CTGGAGACTG GGGGCCTCCT CCCTGGAGAT CCACCCCCAG AACCGACGTC 60
TTGAGGCTGG TGCTGTATCT CACCTTCCTG GGAGCCCCCT GCTACGCCCC AGCTCTGCCG 120
TCCTGCAAGG AGGACGAGTA CCCAGTGGGC TCCGAGTGCT GCCCCAAGTG CAGTCCAGGT 180
TATCGTGTGA AGGAGGCCTG CGGGGAGCTG ACGGGCACAG TGTGTGAACC CTGCCCTCCA 240
GGCACCTACA TTGCCCACCT CAATGGCCTA AGCAAGTGTC TGCAGTGCCA AATGTGTGAC 300
CCAGATATTG GTTCCCCCTG TGACCTCAGG GGAAGAGGTC ACCTGGAGGC TGGTGCCCAC 360
CTGAGTCCAG GCAGACAGAA AGGGGAACCA GACCCAGAGG TGGCCTTTGA GTCACTGAGC 420
GCAGAGCCTG TCCATGCGGC CAACGGCTCT GTCCCCTTGG AGCCTCATGC CAGGCTCAGC 480
ATGGCCAGTG CTCCCTGCGG CCAGGCAGGA CTGCACCTGC GGGACAGGGC TGACGGCACA 540
CCTGGGGGCA GGGCTGAGCC TACAGGGAGG CACAGGGCAG GTGGTCTAGC CATGAACAGA 600
AGAGGAAGCT GGAGTGCTTT CGGGGTTCAT ACATGTAGGC TGGGATTTGG GGCTCACACC 660
TCAACCTGTA TGCCCAGTTC CATGCCCCTC CCCTCTTGTG AAAGCACCTG TCTACTTGGC 720
TGA 723

(2) INFORMATION FOR SEQ ID NO:4:
(i) SEQUENCE CHARACTERISTICS
(A) LENGTH: 240 AMINO ACIDS
(B) TYPE: AMINO ACID
(C) STRANDEDNESS:
(D) TOPOLOGY: LINEAR

(ii) MOLECULE TYPE: PROTEIN

(xi) SEQUENCE DESCRIPTION: SEQ ID NO:4:

(2)    INFORMATION FOR SEQ ID NO:5:

(i)    SEQUENCE CHARACTERISTICS
       (A) LENGTH:  405 BASE PAIRS
       (B) TYPE:  NUCLEIC ACID
       (C) STRANDEDNESS:  SINGLE
       (D) TOPOLOGY:  LINEAR

(ii)   MOLECULE TYPE:  Oligonucleotide

(xi)   SEQUENCE DESCRIPTION:  SEQ ID NO:5:

ATGCTGGGTA CCTCTGGGCA CCTCGTTTGG CTGAGCCAGG GGTTCAGCCT GGCAGGCGCC    60
CTGGCACAGT CCTTGGCCTG TGGATGCTGT CCTGGCCTGT GGATGGTGTC CCGCCCCACG   120
TACCCCTCTC AGCCCCTCCT CTTGGACTCC AGCCATGGCC TGCGCGCGAG CCGGAACTGC   180
TCCAGGACAG AGAACGCCGT GTGTGGCTGC AGCCCAGGCC ACTTCTGCAT CGTCCAGGAC   240
GGGGACCACT GCGCCGCGTG CCGCGCTTAC GCCACCTCCA GCCCGGGCCA GAGGGTGCAG   300
AAGGGAGGCA CCGAGAGTCA GGACACCCTG TGTCAGAACT GCCCCCGGGG ACCTTCTCTC   360
CCAATGGGAC CCTGGAGGAA TGTCAGCACC AGACCAAGTA AGTGA                   405

(2)    INFORMATION FOR SEQ ID NO:6:
  (i)  SEQUENCE CHARACTERISTICS
       (A) LENGTH:  134 AMINO ACIDS
       (B) TYPE:  AMINO ACID
       (C) STRANDEDNESS:
       (D) TOPOLOGY:  LINEAR

  (ii)       MOLECULE TYPE:  PROTEIN

(xi) SEQUENCE DESCRIPTION: SEQ ID NO:6:

Met Leu Gly Thr Ser Gly His Leu Val Trp Leu Ser Gln Gly Phe
                5                   10                  15
Ser Leu Ala Gly Ala Leu Ala Gln Ser Leu Ala Cys Gly Cys Cys
                20                  25                  30
Pro Gly Leu Trp Met Val Ser Arg Pro Thr Tyr Pro Ser Gln Pro
                35                  40                  45
Leu Leu Leu Asp Ser Ser His Gly Leu Arg Ala Ser Arg Asn Cys
                50                  55                  60
Ser Arg Thr Glu Asn Ala Val Cys Gly Cys Ser Pro Gly His Phe
                65                  70                  75
Cys Ile Val Gln Asp Gly Asp His Cys Ala Ala Cys Arg Ala Tyr
                80                  85                  90
Ala Thr Ser Ser Pro Gly Gln Arg Val Gln Lys Gly Gly Thr Glu
                95                  100                 105
Ser Gln Asp Thr Leu Cys Gln Asn Cys Pro Arg Gly Pro Ser Leu
                110                 115                 120
Pro Met Gly Pro Trp Arg Asn Val Ser Thr Arg Pro Ser Lys
                125                 130

(2) INFORMATION FOR SEQ ID NO:7:

(i) SEQUENCE CHARACTERISTICS
(A) LENGTH: 28 BASE PAIRS
(B) TYPE: NUCLEIC ACID
(C) STRANDEDNESS: SINGLE
(D) TOPOLOGY: LINEAR

(ii) MOLECULE TYPE: Oligonucleotide

(xi) SEQUENCE DESCRIPTION: SEQ ID NO:7:

GCGCGGATCC GAGCCTCCTG GAGACTGG 28

(2) INFORMATION FOR SEQ ID NO:8:

(i) SEQUENCE CHARACTERISTICS
(A) LENGTH: 31 BASE PAIRS
(B) TYPE: NUCLEIC ACID
(C) STRANDEDNESS: SINGLE
(D) TOPOLOGY: LINEAR

(ii) MOLECULE TYPE: Oligonucleotide

(xi) SEQUENCE DESCRIPTION: SEQ ID NO:8:

GCGCAAGCTT TCAGTGGTTT GGGCTCCTCC C 31

(2) INFORMATION FOR SEQ ID NO:9:

(i) SEQUENCE CHARACTERISTICS
(A) LENGTH: 34 BASE PAIRS

(B) TYPE: NUCLEIC ACID
(C) STRANDEDNESS: SINGLE
(D) TOPOLOGY: LINEAR

(ii) MOLECULE TYPE: Oligonucleotide

(xi) SEQUENCE DESCRIPTION: SEQ ID NO:9:

GCGCGGATCC ACCATGGAGC CTCCTGGAGA CTGG 34

(2) INFORMATION FOR SEQ ID NO:10:

(i) SEQUENCE CHARACTERISTICS
(A) LENGTH: 31 BASE PAIRS
(B) TYPE: NUCLEIC ACID
(C) STRANDEDNESS: SINGLE
(D) TOPOLOGY: LINEAR

(ii) MOLECULE TYPE: Oligonucleotide

(xi) SEQUENCE DESCRIPTION: SEQ ID NO:10:

GCGCGGTACC TCAGTGGGAG CTGCTGGTCC C 31

(2) INFORMATION FOR SEQ ID NO:11:

(i) SEQUENCE CHARACTERISTICS
(A) LENGTH: 31 BASE PAIRS
(B) TYPE: NUCLEIC ACID
(C) STRANDEDNESS: SINGLE
(D) TOPOLOGY: LINEAR

(ii) MOLECULE TYPE: Oligonucleotide

(xi) SEQUENCE DESCRIPTION: SEQ ID NO:11:

GCGCGGTACC TCAGTGGTTT GGGCTCCTCC C 31

(2) INFORMATION FOR SEQ ID NO:12:

(i) SEQUENCE CHARACTERISTICS
(A) LENGTH: 34 BASE PAIRS
(B) TYPE: NUCLEIC ACID
(C) STRANDEDNESS: SINGLE
(D) TOPOLOGY: LINEAR

(ii) MOLECULE TYPE: Oligonucleotide

(xi) SEQUENCE DESCRIPTION: SEQ ID NO:12:

GCGCGGATCC ACCATGGAGC CTCCTGGAGA CTGG 34

(2) INFORMATION FOR SEQ ID NO:13:

(i)      SEQUENCE CHARACTERISTICS
         (A) LENGTH:  58 BASE PAIRS
         (B) TYPE:  NUCLEIC ACID
         (C) STRANDEDNESS:  SINGLE
         (D) TOPOLOGY:  LINEAR

(ii)     MOLECULE TYPE:  Oligonucleotide

(xi)     SEQUENCE DESCRIPTION:  SEQ ID NO:13:

GCGCTCTAGA TCAAGCGTAG TCTGGGACGT CGTATGGGTA GTGGTTTGGG CTCCTCCC     58

## Description

This invention relates to newly identified polynucleotides, polypeptides encoded by such polynucleotides, the use of such polynucleotides and polypeptides, as well as the production of such polynucleotides and polypeptides. The polypeptides of the present invention have been putatively identified as members of the Nerve Growth Factor/Tumor Necrosis Factor receptor family. More particularly, the polypeptides have been putatively identified as a Tumor Necrosis Factor and two different splice variants thereof. The Tumor Necrosis Factor will hereinafter be referred to as "TNF" Receptor" and the splice variants will be referred to as "TNF-SV1" and "TNF-SV2," respectively. The invention also relates to inhibiting the signal generated by such polypeptides.

Human tumor necrosis factors α (TNF-α) and β (TNF-β or lymphotoxin) are related members of a broad class of polypeptide mediators, which includes the interferons, interleukins and growth factors, collectively called cytokines (Beutler, B. and Cerami, A., Annu. Rev. Immunol., 7:625-655 (1989)).

Tumor necrosis factor (TNF-β and TNF-β) was originally discovered as a result of its anti-tumor activity, however, now it is recognized as a pleiotropic cytokine playing important roles in a host of biological processes and pathologies. To date, there are eight known members of the TNF-related cytokine family, TNF-α, TNF-β (lymphotoxin-α) , LT-β, and ligands for the Fas receptor, CD30, CD27, CD40 and 4-1BB receptors. These proteins have conserved C-tertninal sequences and variable N-terminal sequences which are often used as membrane anchors, with the exception of TNF-β. Both TNF-α and TNF-β function as homotrimers when they bind to TNF receptors.

TNF is produced by a number of cell types, including monocytes, fibroblasts, T cells, natural killer (NK) cells and predominately by activated machrophages. TNF-α has been reported to have a role in the rapid necrosis of tumors, immunostimulation, autoimmune disease including arthritis, graft rejection, producing an anti-viral response, septic shock, cerebral malaria, cytotoxicity, protection against deleterious effects of ionizing radiation produced during a course of chemotherapy, such as denaturation of enzymes, lipid peroxidation and DNA damage (Nata et al, J. Immunol . 136(7):2483 (1987)), growth regulation, vascular endothelium effects and metabolic effects. TNF-α also triggers endothelial cells to secrete various factors, including PAI-1, IL-1, GM-CSF and IL-6 to promote cell proliferation. In addition, TNF-α up-regulates various cell adhesion molecules such as E-Selectin, ICAM-1 and VCAM-1. TNF-α and the Fas ligand have also been shown to induce programmed cell death.

A related molecule, lymphotoxin (LT, also referred to as TNF-β, which is produced by activated lymphocytes shows a similar but not identical spectrum of biological activities as TNF. Two different types of LT have been found, LT-α and LT-β. LT-α has many activities, including tumor necrosis, induction of an antiviral state, activation of polymorphonuclear leukocytes, induction of class I major histocompatibility complex antigens on endothelial cells, induction of adhesion molecules on endothelium and growth hormone stimulation (Ruddle, N. and Homer, R., Prog. Allergy, 40:162-182 (1988)).

The first step in the induction of the various cellular responses mediated by TNF or LT is their binding to specific cell surface receptors. Two distinct TNF receptors of approximately 55-KDa (TNF-R1) and 75-KDa (TNF-R2) have been identified (Hohman, H.P. et al., J. Biol. Chem., 264:14927-14934 (1989)), and human and mouse cDNAs corresponding to both receptor types have been isolated and characterized (Loetscher, H. et al., Cell, 61:351 (1990)). Both TNF-Rs share the typical structure of cell surface receptors including extracellular, transmembrane and intracellular regions.

These molecules exist not only in cell bound forms, but also in soluble forms, consisting of the cleaved extra-cellular domains of the intact receptors (Nophar et al., EMBO Journal, 9 (10):3269-76 (1990)). The extracellular domains of TNF-R1 and TNF-R2 share 28% identity and are characterized by four repeated cysteine-rich motifs with significant intersubunit sequence homology. The majority of cell types and tissues appear to express both TNF receptors and both receptors are active in signal transduction, however, they are able to mediate distinct cellular responses. Further, TNF-R2 was shown to exclusively mediate human T cell proliferation by TNF as shown in PCT Publication No. WO 94/09137.

TNF-R1 dependent responses include accumulation of C-FOS, IL-6, and manganese superoxide dismutase mRNA, prostaglandin E2 synthesis, IL-2 receptor and MHC class I and II cell surface antigen expression, growth inhibition, and cytotoxicity.

TNF-R1 triggers second messenger systems such as phospholipase A₂, protein kinase C, phosphatidylcholine-specific phospholipase C and sphingomyelinase (Pfefferk et al., Cell, 73:457-467 (1993)).

The TNF receptor and the two splice variants belong to a family of receptors and antigens known as the Nerve Growth Factor/Tumor Necrosis Factor Family. This family shows highly conserved cysteine residues and includes the low affinity NGF receptor, which plays an important role in the regulation of growth and differentiation of nerve cells, the FAS receptor also called APO, a receptor which is involved in signalling for apoptosis and which, based on a study with mice deficient in its function, seems to play an important role in the etiology of a lupus-like disease, the TNF-R1, the B cell antigen CD40, and the T cell activation antigen CD27.

The receptor polypeptides of the present invention have been putatively identified as members of this family. The receptor polypeptides of the present invention show significant amino acid sequence homology to the type 2 human tumor necrosis factor receptor.

In accordance with one aspect of the present invention, there are provided novel mature polypeptides, as well as fragments, analogs and derivatives thereof. The polypeptides of the present invention are of human origin.

In accordance with another aspect of the present invention, there are provided isolated nucleic acid molecules encoding such polypeptides, including mRNAs, DNAs, cDNAs, genomic DNA as well as antisense analogs thereof and biologically active and diagnostically or therapeutically useful fragments thereof.

In accordance with yet a further aspect of the present invention, there are provided processes for producing such polypeptides by recombinant techniques which comprises culturing recombinant prokaryotic and/or eukaryotic host cells, containing a nucleic acid sequence encoding a polypeptide of the present invention, under conditions promoting expression of said protein and subsequent recovery of said protein.

In accordance with yet a further aspect of the present invention, there are provided processes for utilizing such polypeptides, or polynucleotides encoding such polypeptides to screen for receptor antagonists and/or agonists and/or receptor ligands.

In accordance with yet a further aspect of the present invention, there are provided nucleic acid probes comprising nucleic acid molecules of sufficient length to specifically hybridize to the polynucleotide sequences of the present invention.

In accordance with still another aspect of the present invention, there are provided processes of using such agonists for treating conditions related to insufficient activity of the polypeptides of the present invention, for example, to inhibit tumor growth, to stimulate human cellular proliferation, e.g., T-cell proliferation, to regulate the immune response and antiviral responses, to protect against the effects of ionizing radiation, to protect against chlamidiae infection, to regulate growth and to treat immunodeficiencies such as is found in HIV.

In accordance with another aspect of the present invention, there are provided processes of using such antagonists for treating conditions associated with over-expression of the polypeptides of the present invention, for example, for treating T-cell mediated autoimmune diseases such as AIDS and arthritis, septic shock, cerebral malaria, graft rejection, cytotoxicity, cachexia, apoptosis and inflammation.

These and other aspects of the present invention should be apparent to those skilled in the art from the teachings herein.

The following drawings are illustrative of embodiments of the invention and are not meant to limit the scope of the invention as encompassed by the claims.
Figure 1 shows the cDNA sequence and corresponding deduced amino acid sequence of the TNF receptor of the present invention. The initial 38 amino acids represent the putative leader sequence and are underlined. The standard one-letter abbreviations for amino acids are used.

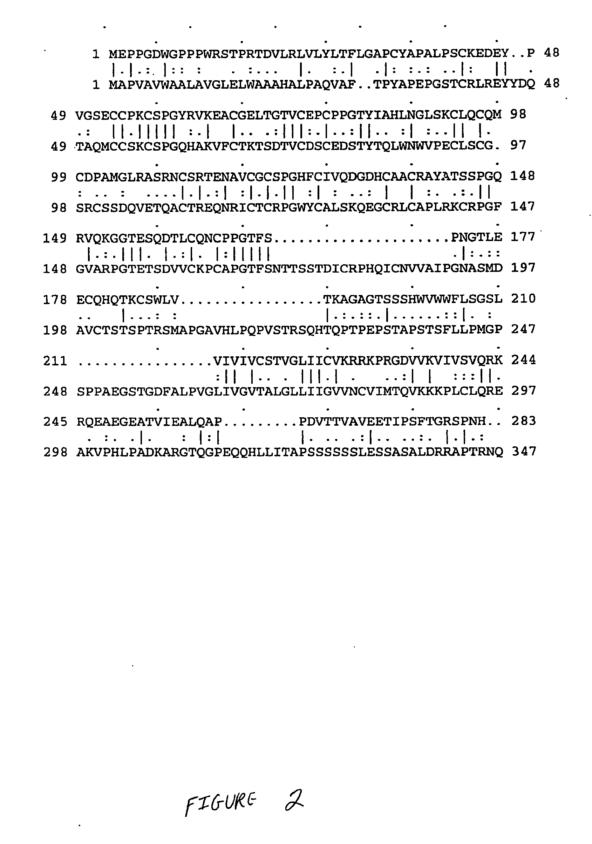

Figure 2 illustrates an amino acid sequence alignment of the TNF receptor polypeptide of the present invention (upper line) and the human type 2 TNF receptor (lower line).
Figure 3 illustrates the cDNA sequence and corresponding deduced amino acid sequence of the TNF receptor-splice variant 1 of the present invention. The initial 38 amino acids represent the putative leader sequence. The standard one-letter abbreviations for amino acids are used.

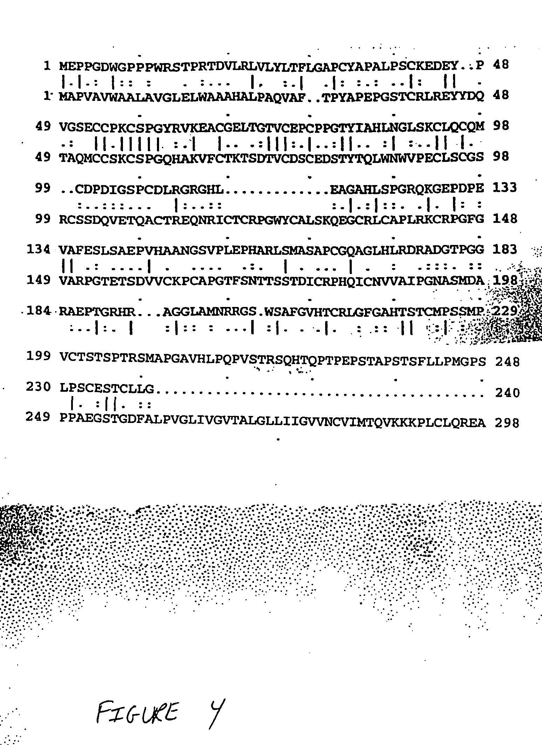

Figure 4 illustrates an amino acid sequence alignment of the TNF-SV1 of the present invention (upper line) and the human type 2 TNF receptor (lower line).
Figure 5 illustrates the cDNA sequence in corresponding deduced amino acid sequence of the TNF-SV2 receptor of the present invention. The standard one-letter abbreviations for amino acids are used.

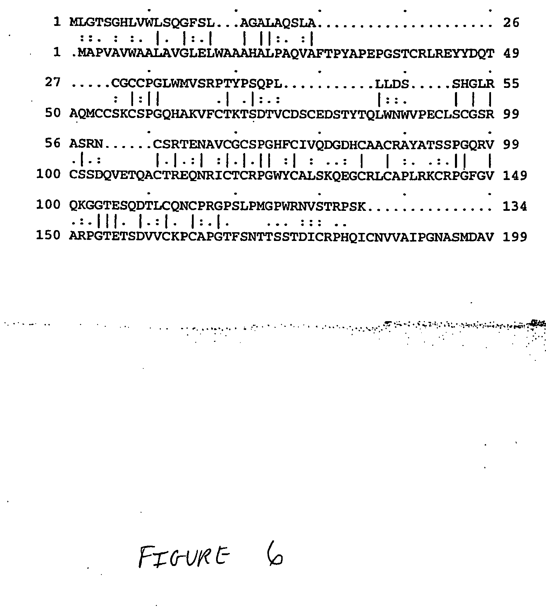

Figure 6 illustrates an amino acid sequence alignment of the TNF-SV2 of the polypeptide of the present invention (upper line) and the human type 2 TNF receptor (lower line).

This invention relates to TNF receptor, the amino acid sequence of which is set forth in SEQ ID NO:2. The invention also relates to TNF-SV1, the amino acid sequence of which is set forth in SEQ ID NO:4 and TNF-SV2, the amino acid sequence of which is set forth in SEQ ID NO:6.

The polypeptides of this invention may be membrane bound of may be in a soluble circulating form.

This invention also relates to soluble forms of the polypeptides of the present invention. Soluble peptides are defined by the amino acid sequence wherein the sequence comprises the polypeptide sequence lacking the transmembrane domain. Such soluble peptides may include any number leader requences at the 5' end. Such a sequence would allow the peptides to be expressed in a eukaryotic system (see, e.g., Ernst et al., U.S. Patent No. 5,082,783 (1992)).

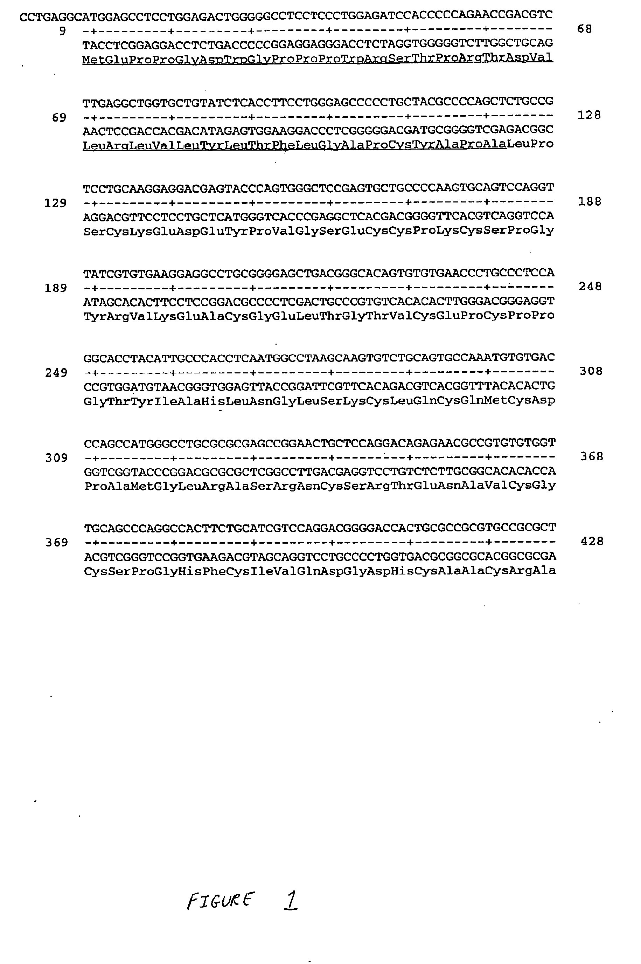

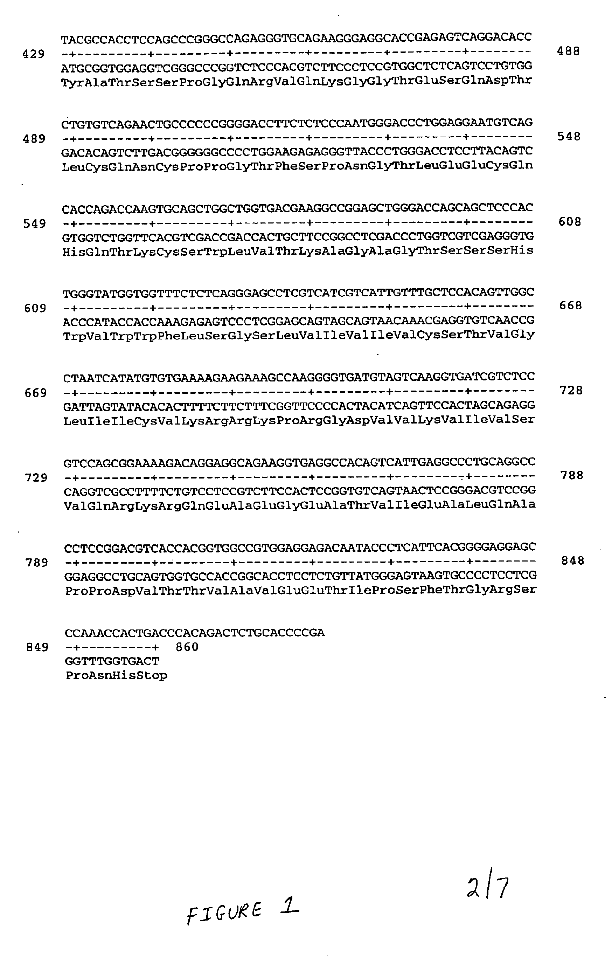

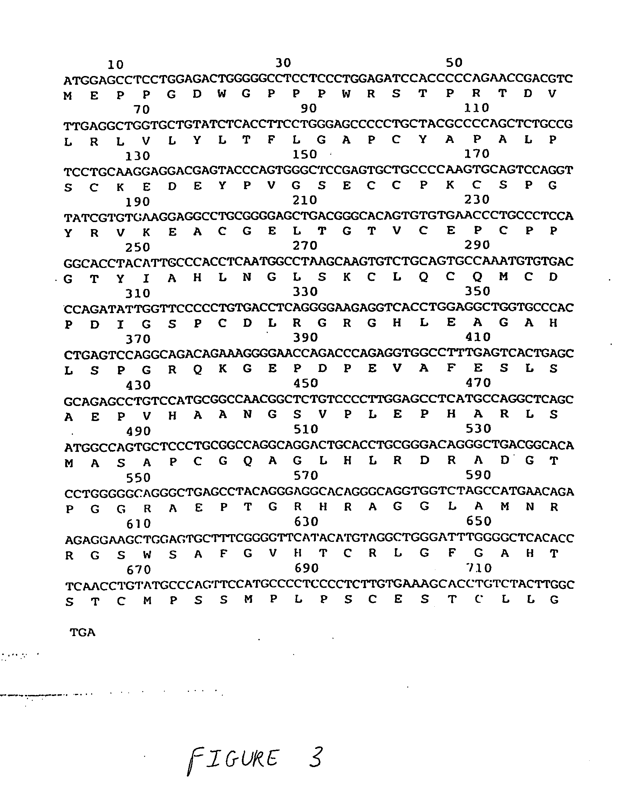

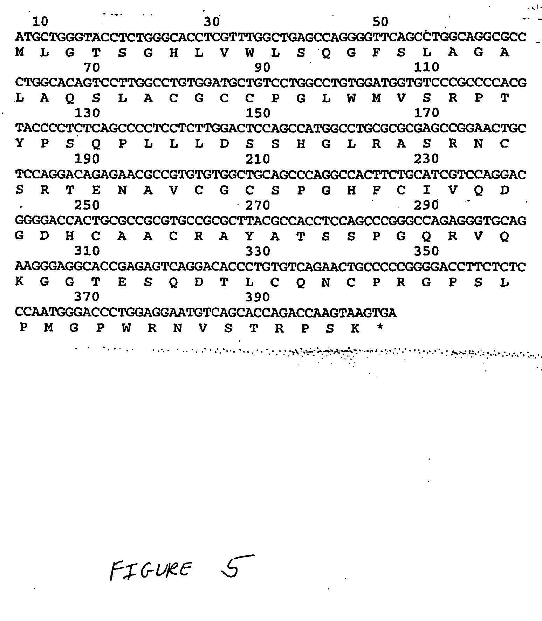

In accordance with an aspect of the present invention, there are provided isolated nucleic acids (polynucleotides) which encode for the mature polypeptides having the deduced amino acid sequence of Figures 1, 3 and 5 (SEQ ID No. 2, 4 and 6) or for the mature polypeptide encoded by the cDNA of the clone (s) deposited as ATCC Deposit No. 97059 (TNF Receptor), ATCC Deposit No. 97058 (TNF-SV1) and ATCC Deposit No. 97057 (TNF-SV2) on February 13, 1995.

A polynucleotide encoding the TNF receptor polypeptide of the present invention may be obtained from human lymphocytes, tonsils and fetal lung. The polynucleotide of this invention was discovered in a cDNA library derived from a human lymphocyte. It is structurally related to the human NGF/TNF receptor family. It contains an open reading frame encoding a protein of 283 amino acid residues of which approximately the first 38 amino acids residues are the putative leader sequence such that the mature protein comprises 265 amino acids. The protein exhibits the highest degree of homology to a human type 2 TNF receptor with 32% identity and 44% similarity over a 117 amino acid stretch.

A polynucleotide encoding the TNF-SV1 polypeptide of the present invention may be obtained from human lymphocytes, tonsils and fetal lung. The polynucleotide of this invention was discovered in a cDNA library derived from a human fetal heart. It is structurally related to the human NGF/TNF receptor family. It contains an open reading frame encoding a protein of 240 amino acid residues of which approximately the first 38 amino acids residues are the putative leader sequence such that the mature protein comprises 202 amino acids. The protein exhibits the highest degree of homology to a human type 2 TNF receptor with 21% identity and 42% similarity over the entire amino acid sequence.

A polynucleotide encoding the TNF-SV2 polypeptide of the present invention may be obtained from human lymphocytes, tonsils and fetal lung. The polynucleotide of this invention was discovered in a cDNA library derived from a human stimulated monocyte. It is structurally related to the human NGF/TNF receptor family. It contains an open reading frame encoding a protein of 134 amino acid residues. The protein exhibits the highest degree of homology to a human type 2 TNF receptor with 27% identity and 48% similarity over the entire amino acid sequence.

The polypeptides of the present invention may exist as a membrane bound receptor having a transmembrane region and an intra- and extracellular region or it may exist in soluble form wherein the transmembrane domain is lacking. The polypeptides of the present invention may bind TNF and lymphotoxin molecules.

In accordance with an aspect of the present invention there are provided a polynucleotides which may be in the form of RNA or in the form of DNA, which DNA includes cDNΔ, genomic DNA, and synthetic DNA. The DNA may be doublestranded or single-stranded, and if single stranded may be the coding strand or non-coding (anti-sense) strand. The coding sequences which encode the mature polypeptides may be identical to the coding sequences shown in Figure 1, 3 and 5 (SEQ ID No. 1, 3 and 5) or that of the deposited clone(s) or may be a different coding sequence which coding sequence, as a result of the redundancy or degeneracy of the genetic code, encodes the same mature polypeptide as the DNA of Figure 1, 3 and 5 (SEQ ID No. 1, 3 and 5) or the deposited cDNA(s).

The polynucleotides which encode for the mature polypeptides of Figure 1, 3 and 5 (SEQ ID No. 2, 4 and 6) or for the mature polypeptides encoded by the deposited cDNA(s) may include: only the coding sequence for the mature polypeptide; the coding sequence for the mature polypeptide and additional coding sequence such as a leader or secretory sequence or a proprotein sequence; the coding sequence for the mature polypeptide (and optionally additional coding sequence) and non-coding sequence, such as introns or non-coding sequence 5' and/or 3' of the coding sequence for the mature polypeptide.

Thus, the term "polynucleotide encoding a polypeptide" encompasses a polynucleotide which includes only coding sequence for the polypeptide as well as a polynucleotide which includes additional coding and/or non-coding sequence.

The present invention further relates to variants of the hereinabove described polynucleotides which encode for fragments, analogs and derivatives of the polypeptide having the deduced amino acid sequence of Figure 1, 3 and 5 (SEQ ID No. 2, 4 and 6) or the polypeptides encoded by the cDNA(s) of the deposited clone(s). The variants of the polynucleotides may be naturally occurring allelic variants of the polynucleotides or non-naturally occurring variants of the polynucleotides.

Thus, the present invention includes polynucleotides encoding the same mature polypeptides as shown in Figure 1, 3 and 5 (SEQ ID No. 2, 4 and 6) or the same mature polypeptides encoded by the cDNA(s) of the deposited clone(s) as well as variants of such polynucleotides which variants encode for a fragment, derivative or analog of the polypeptide of Figure 1, 3 and 5 (SEQ ID No. 2, 4 and 6) or the polypeptides encoded by the cDNA(s) of the deposited clone(s). Such nucleotide variants include deletion variants, substitution variants and addition or insertion variants.

As hereinabove indicated, the polynucleotides may have a coding sequence which is a naturally occurring allelic variant of the coding sequences shown in Figure 1, 3 and 5 (SEQ ID No. 1, 3 and 5) or of the coding sequences of the deposited clone(s). As known in the art, an allelic variant is an alternate form of a polynucleotide sequence which may have a substitution, deletion or addition of one or more nucleotides, which does not substantially alter the function of the encoded polypeptide.

The polynucleotides of the present invention may also have the coding sequence fused in frame to a marker sequence which allows for purification of the polypeptide of the present invention. The marker sequence may be a hexa-histidine tag supplied by a pQE-9 vector to provide for purification of the mature polypeptide fused to the marker in the case of a bacterial host, or, for example, the marker sequence may be a hemagglutinin (HA) tag when a mammalian host, e.g. COS-7 cells, is used. The HA tag corresponds to an epitope derived from the influenza hemagglutinin protein (Wilson, I., et al., Cell, 37:767 (1984)).

The present invention further relates to polynucleotides which hybridize to the hereinabove-described sequences if there is at least 50% and preferably 70% identity between the sequences. The present invention particularly relates to polynucleotides which hybridize under stringent conditions to the hereinabove-described polynucleotides. As herein used, the term "stringent conditions" means hybridization will occur only if there is at least 95% and preferably at least 97% identity between the sequences. The polynucleotides which hybridize to the hereinabove described polynucleotides in a preferred embodiment encode polypeptides which retain substantially the same biological function or activity as the mature polypeptides encoded by the cDNA(s) of Figure 1, 3 and 5 (SEQ ID No. 1, 3 and 5) or the deposited cDNA(s).

The deposit(s) referred to herein will be maintained under the terms of the Budapest Treaty on the International Recognition of the Deposit of Micro-organisms for purposes of Patent Procedure. These deposits are provided merely as convenience to those of skill in the art and are not an admission that a deposit is required under 35 U.S.C. §112. The sequence of the polynucleotides contained in the deposited materials, as well as the amino acid sequence of the polypeptides encoded thereby, are incorporated herein by reference and are controlling in the event of any conflict with any description of sequences herein. A license may be required to make, use or sell the deposited materials, and no such license is hereby granted.

The present invention further relates to polypeptides which have the deduced amino acid sequences of Figure 1, 3 and 5 (SEQ ID No. 2, 4 and 6) or which have the amino acid sequences encoded by the deposited cDNA(s), as well as fragments, analogs and derivatives of such polypeptides.

The terms "fragment," "derivative" and "analog" when referring to the polypeptides of Figures 1, 3 and 5 (SEQ ID No:2, 4 and 6) or that encoded by the deposited cDNA(s), means a polypeptide which retains essentially the same biological function or activity as such polypeptide. Thus, an analog includes a proprotein which can be activated by cleavage of the proprotein portion to produce an active mature polypeptide.

The polypeptides of the present invention may be recombinant polypeptides, natural polypeptides or synthetic polypeptides, preferably recombinant polypeptides.

The fragment, derivative or analog of the polypeptide of Figure 1, 3 and 5 (SEQ ID NO:2, 4 and 6) or that encoded by the deposited cDNA may be (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which the mature polypeptide is fused with another compound, such as a compound to increase the half-life of the polypeptide (for example, polyethylene glycol), or (iv) one in which the additional amino acids are fused to the mature polypeptide, such as an Fc fusion region peptide or leader or secretory sequence or a sequence which is employed for purification of the mature polypeptide or a proprotein sequence. Such fragments, derivatives and analogs are deemed to be within the scope of those skilled in the art from the teachings herein.

The polypeptides and polynucleotides of the present invention are preferably provided in an isolated form, and preferably are purified to homogeneity.

The term "gene" means the segment of DNA involved in producing a polypeptide chain; it includes regions preceding and following the coding region (leader and trailer) as well as intervening sequences (introns) between individual coding segments (exons).

The term "isolated" means that the material is removed from its original environment (e.g., the natural environment if it is naturally occurring). For example, a naturally-occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition, and still be isolated in that such vector or composition is not part of its natural environment.

The present invention also relates to vectors which include polynucleotides of the present invention, host cells which are genetically engineered with vectors of the invention and the production of polypeptides of the invention by recombinant techniques.

Host cells are genetically engineered (transduced or transformed or transfected) with the vectors of this invention which may be, for example, a cloning vector or an expression vector. The vector may be, for example, in the form of a plasmid, a viral particle, a phage, etc. The engineered host cells can be cultured in conventional nutrient media modified as appropriate for activating promoters, selecting transformants or amplifying the NGF/TNF receptor genes. The culture conditions, such as temperature, pH and the like, are those previously used with the host cell selected for expression, and will be apparent to the ordinarily skilled artisan.

The polynucleotides of the present invention may be employed for producing polypeptides by recombinant techniques. Thus, for example, the polynucleotide may be included in any one of a variety of expression vectors for expressing a polypeptide. Such vectors include chromosomal, nonchromosomal and synthetic DNA sequences, e.g., derivatives of SV40; bacterial plasmids; phage DNA; baculovirus; yeast plasmids; vectors derived from combinations of plasmids and phage DNA, viral DNA such as vaccinia, adenovirus, fowl pox virus, and pseudorabies.

The appropriate DNA sequence may be inserted into the vector by a variety of procedures. In general, the DNA sequence is inserted into an appropriate restriction endonuclease site(s) by procedures known in the art. Such procedures and others are deemed to be within the scope of those skilled in the art.

The DNA sequence in the expression vector is operatively linked to an appropriate expression control sequence(s) (promoter) to direct mRNA synthesis. As representative examples of such promoters, there may be mentioned: LTR or SV40 promoter, the E. coli. lac or trp, the phage lambda P_{L} promoter and other promoters known to control expression of genes in prokaryotic or eukaryotic cells or their viruses. The expression vector also contains a ribosome binding site for translation initiation and a transcription terminator. The vector may also include appropriate sequences for amplifying expression.

In addition, the expression vectors preferably contain one or more selectable marker genes to provide a phenotypic trait for selection of transformed host cells such as dihydrofolate reductase or neomycin resistance for eukaryotic cell culture, or such as tetracycline or ampicillin resistance in E. coli.

The vector containing the appropriate DNA sequence as hereinabove described, as well as an appropriate promoter or control sequence, may be employed to transform an appropriate host to permit the host to express the protein.

As representative examples of appropriate hosts, there may be mentioned: bacterial cells, such as E. coli, Streptomyces, Salmonella typhimurium; fungal cells, such as yeast; insect cells such as Drosophila S2 and Spodoptera Sf9; animal cells such as CHO, COS or Bowes melanoma; adenoviruses; plant cells, etc. The selection of an appropriate host is deemed to be within the scope of those skilled in the art from the teachings herein.

More particularly, the present invention also includes recombinant constructs comprising one or more of the sequences as broadly described above. The constructs comprise a vector, such as a plasmid or viral vector, into which a sequence of the invention has been inserted, in a forward or reverse orientation. In a preferred aspect of this embodiment, the construct further comprises regulatory sequences, including, for example, a promoter, operably linked to the sequence. Large numbers of suitable vectors and promoters are known to those of skill in the art, and are commercially available. The following vectors are provided by way of example. Bacterial: pQE70, pQE60, pQE-9 (Qiagen), pBS, pD10, phagescript, psiX174, pbluescript SK, pbsks, pNH8A, pNH16a, pNH18A, pNH46A (Stratagene); pTRC99a, pKK223-3, pKK233-3, pDR540, pRIT5 (Pharmacia). Eukaryotic: pWLNEO, pSV2CAT, pOG44, pXT1, pSG (Stratagene) pSVK3, pBPV, pMSG, pSVL (Pharmacia). However, any other plasmid or vector may be used as long as they are replicable and viable in the host.

Promoter regions can be selected from any desired gene using CAT (chloramphenicol transferase) vectors or other vectors with selectable markers. Two appropriate vectors are PKK232-8 and PCM7. Particular named bacterial promoters include lacI, lacZ, T3, T7, gpt, lambda P_{R}, P_{I}. and trp. Eukaryotic promoters include CMV immediate early, HSV thymidine kinase, early and late SV40, LTRs from retrovirus, and mouse metallothionein-I. Selection of the appropriate vector and promoter is well within the level of ordinary skill in the art.

In a further embodiment, the present invention relates to host cells containing the above-described constructs. The host cell can be a higher eukaryotic cell, such as a mammalian cell, or a lower eukaryotic cell, such as a yeast cell, or the host cell can be a prokaryotic cell, such as a bacterial cell. Introduction of the construct into the host cell can be effected by calcium phosphate transfection, DEAE-Dextran mediated transfection, or electroporation (Davis, L., Dibner, M., Battey, I., Basic Methods in Molecular Biology, (1986)).

The constructs in host cells can be used in a conventional manner to produce the gene product encoded by the recombinant sequence. Alternatively, the polypeptides of the invention can be synthetically produced by conventional peptide synthesizers.

Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook, et al., Molecular Cloning: A Laboratory Manual, Second Edition, Cold Spring Harbor, N.Y., (1989), the disclosure of which is hereby incorporated by reference.

Transcription of the DNA encoding the polypeptides of the present invention by higher eukaryotes is increased by inserting an enhancer sequence into the vector. Enhancers are cis-acting elements of DNA, usually about from 10 to 300 bp that act on a promoter to increase its transcription. Examples include the SV40 enhancer on the late side of the replication origin bp 100 to 270, a cytomegalovirus early promoter enhancer, the polyoma enhancer on the late side of the replication origin, and adenovirus enhancers.

Generally, recombinant expression vectors will include origins of replication and selectable markers permitting transformation of the host cell, e.g., the ampicillin resistance gene of E. coli and S. cerevisiae TRP1 gene, and a promoter derived from a highly-expressed gene to direct transcription of a downstream structural sequence. Such promoters can be derived from operons encoding glycolytic enzymes such as 3-phosphoglycerate kinase (PGK), α-factor, acid phosphatase, or heat shock proteins, among others. The heterologous structural sequence is assembled in appropriate phase with translation initiation and termination sequences, and preferably, a leader sequence capable of directing secretion of the translated protein or part of the translated protein into the periplasmic space or extracellular medium. Optionally, the heterologous sequence can encode a fusion protein including an N-terminal identification peptide imparting desired characteristics, e.g., stabilization or simplified purification of expressed recombinant product.

Useful expression vectors for bacterial use are constructed by inserting a structural DNA sequence encoding a desired protein together with suitable translation initiation and termination signals in operable reading phase with a functional promoter. The vector will comprise one or more phenotypic selectable markers and an origin of replication to ensure maintenance of the vector and to, if desirable, provide amplification within the host. Suitable prokaryotic hosts for transformation include E. coli, Bacillus subtilis, Salmonella typhimurium and various species within the genera Pseudomonas, Streptomyces, and Staphylococcus, although others may also be employed as a matter of choice.

As a representative but nonlimiting example, useful expression vectors for bacterial use can comprise a selectable marker and bacterial origin of replication derived from commercially available plasmids comprising genetic elements of the well known cloning vector pBR322 (ATCC 37017). Such commercial vectors include, for example, pKK223-3 (Pharmacia Fine Chemicals, Uppsala, Sweden) and GEM1 (Promega Biotec, Madison, WI, USA). These pBR322 "backbone" sections are combined with an appropriate promoter and the structural sequence to be expressed.

Following transformation of a suitable host strain and growth of the host strain to an appropriate cell density, the selected promoter is induced by appropriate means (e.g., temperature shift or chemical induction) and cells are cultured for an additional period.

Cells are typically harvested by centrifugation, disrupted by physical or chemical means, and the resulting crude extract retained for further purification.

Microbial cells employed in expression of proteins can be disrupted by any convenient method, including freeze-thaw cycling, sonication, mechanical disruption, or use of cell lysing agents, such methods are well know to those skilled in the art.

Various mammalian cell culture systems can also be employed to express recombinant protein. Examples of mammalian expression systems include the COS-7 lines of monkey kidney fibroblasts, described by Gluzman, Cell, 23:175 (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 splice, and polyadenylation sites may be used to provide the required nontranscribed genetic elements.

The polypeptides of the present invention can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Protein refolding steps can be used, as necessary, in completing configuration of the mature protein. Finally, high performance liquid chromatography (HPLC) can be employed for final purification steps.

The polypeptides of the present invention may be a naturally purified product, or a product of chemical synthetic procedures, or produced by recombinant techniques from a prokaryotic or eukaryotic host (for example, by bacterial, yeast, higher plant, insect and mammalian cells in culture). Depending upon the host employed in a recombinant production procedure, the polypeptides of the present invention may be glycosylated or may be non-glycosylated. Polypeptides of the invention may also include an initial methionine amino acid residue.

Fragments of the full length genes of the present invention may be used as hybridization probes for cDNA libraries to isolate genes which have a high sequence similarity to the genes of the present invention or which have similar biological activity. Probes of this this type, preferably have at least 30 bases and generally do not exceed 50 bases, although they may have a greater number of bases. The probes may also be used to identify cDNA clone(s) corresponding to a full length transcript and a genomic clone or clones that contain the complete gene including regulatory and promotor regions, exons, and introns. An example of a screen comprises isolating the coding region of the genes of the present invention by using the known DNA sequence to synthesize an oligonucleotide probe. Labeled oligonucleotides having a sequence complementary to that of the genes of the present invention are used to screen a library of human cDNA, genomic DNA or mRNA to determine which members of the library the probe hybridizes to.

The polypeptides of the present invention may be employed for identifying ligands which can bind to the receptor polypeptides which comprises transfecting a cell population with the appropriate vector expressing the receptors, such that the cell will now express the receptor. A suitable response system is obtained by transfection of the DNA into a suitable host containing the desired second messenger pathways including cAMP, ion channels, phosphoinositide kinase, or calcium response. Such a transfection system provides a response system to analyze the activity of various ligands exposed to the cell. Ligands chosen could be identified through a rational approach by taking known ligands that interact with similar types of receptors or using small molecules, cell supernatants or extracts or natural products.

Alternatively, a determination may be made whether a ligand not known to be capable of binding to a receptor polypeptide of the present invention can bind thereto comprising contacting a mammalian cell comprising an isolated molecule encoding a receptor polypeptide of the present invention with a ligand under conditions permitting binding of ligands known to bind to a TNF receptor, detecting the presence of any of the ligand bound to a receptor polypeptide of the present invention, and thereby determining whether such ligands bind thereto.

Also, soluble forms of the polypeptides of the present invention may be utilized in the above assay where they are secreted in to the extra-cellular medium and contacted with ligands to determine which will bind to the soluble form of the particular receptor polypeptide.

This invention also provides a method of screening compounds to identify compounds which interact with, and activate or block the activation of the receptor polypeptides of the present invention on the surface of a cell which comprises contacting a mammalian cell comprising an isolated DNA molecule encoding and expressing a polypeptide of the present invention with a plurality of compounds, determining those which activate or block the activation of the polypeptides of the present invention in the mammalian cell using a bioassay such as a second messenger assay, and thereby identifying compounds which specifically interact with, and activate or block the activation of the polypeptides of the present invention. The soluble form of the receptor polypeptides of the present invention may also be employed to identify agonists and antagonists in a thymocyte proliferation assay.

A thymocyte proliferation assay may be employed to identify both ligands and potential drug candidates. For example, thymus cells are disaggregated from tissue and grown in culture medium. Incorporation of DNA prescursbrs such as ³H-thymidine or 5-bromo-2'-deoxyuridine (BrdU) is monitored as a parameter for DNA synthesis and cellular proliferation. Cells which have incorporated BrdU into DNA can be detected using a monoclonal antibody against BrdU and measured by an enzyme or fluorochrome-conjugated second antibody. The reaction is quantitated by fluorimetry or by spectrophotometry. Two control wells and an experimental well are set up as above and TNF-β is added to all wells while soluble receptor polypeptides of the present invention are added individually to the second control wells, with the experimental well containing a compound to be screened. The ability of the compound to be screened to inhibit the above interaction may then be quantified.

The present invention further relates to a diagnostic assay which detects altered levels of soluble forms of the polypeptides of the present invention where an elevated level of the soluble form of the polypeptides in a sample derived from a host is indicative of certain diseases. Assays available to detect levels of soluble polypeptides are well known to those of skill in the art, for example, radioimmunoassays, competitive-binding assays, Western blot analysis, and preferably an ELISA assay may be employed.

An ELISA assay initially comprises preparing an antibody specific to an antigen specific to a receptor polypeptide of the present invention, preferably a monoclonal antibody. In addition a reporter antibody is prepared against the monoclonal antibody. To the reporter antibody is attached a detectable reagent such as radioactivity, fluorescence or in this example, a horseradish peroxidase enzyme. A sample is now removed from a host and incubated on a solid support, e.g., a polystyrene dish, that binds the proteins in the sample. Any free protein binding sites on the dish are then covered by incubating with a non-specific protein like BSA. Next, the monoclonal antibody is incubated in the dish during which time the monoclonal antibodies attach to any polypeptides of the present invention which are attached to the polystyrene dish. All unbound monoclonal antibody is washed out with buffer. The reporter antibody linked to horseradish peroxidase is now placed in the dish resulting in binding of the reporter antibody to any monoclonal antibody bound to a polypeptide of the present invention. Unattached reporter antibody is then washed out. Peroxidase substrates are then added to the dish and the amount of color developed in a given time period is a measurement of the amount of protein of interest which is present in a given volume of patient sample when compared against a standard curve.

A competition assay may be employed wherein antibodies specific to any one of the polypeptides of the present invention are attached to a solid support. A labeled polypeptide of the present invention and a sample derived from a host are then passed over the solid support and the amount of label detected attached to the solid support can be correlated to a quantity of a polypeptide of the present invention in the sample.

The invention also contemplates the use of a gene of the present invention as a diagnostic. For example, if a mutation is present in one of the genes of the present invention, conditions would result from a lack of production of the receptor polypeptides of the present invention. Further, mutations which enhance receptor polypeptide activity would lead to diseases associated with an over-expression of the receptor polypeptide, e.g., endotoxic shock. Mutations in the genes can be detected by comparing the sequence of the defective gene with that of a normal one. Subsequently one can verify that a mutant gene is associated with a disease condition or the susceptibility to a disease condition. That is, a mutant gene which leads to the underexpression of the receptor polypeptides of the present invention would be associated with an inability of TNF to inhibit tumor growth.

Individuals carrying mutations in the genes of the present invention may be detected at the DNA level by a variety of techniques. Nucleic acids used for diagnosis may be obtained from a patient's cells, such as from blood, urine, saliva and tissue biopsy among other tissues. The genomic DNA may be used directly for detection or may be amplified enzymatically by using PCR (Saiki et al., Nature, 324:163-166 (1986)) prior to analysis. RNA or cDNA may also be used for the same purpose. As an example, PCR primers complementary to the nucleic acid of the instant invention can be used to identify and analyze mutations in the human genes of the present invention. For example, deletions and insertions can be detected by a change in the size of the amplified product in comparison to the normal genotype. Point mutations can be identified by hybridizing amplified DNA to radiolabeled RNA or alternatively, radiolabeled antisense DNA sequences of the present invention. Perfectly matched sequences can be distinguished from mismatched duplexes by RNase A digestion or by differences in melting temperatures. Such a diagnostic would be particularly useful for prenatal or even neonatal testing.

Sequence differences between the reference gene and "mutants" may be revealed by the direct DNA sequencing method. In addition, cloned DNA segments may be used as probes to detect specific DNA segments. The sensitivity of this method is greatly enhanced when combined with PCR. For example, a sequencing primary used with double stranded PCR product or a single stranded template molecule generated by a modified PCR product. The sequence determination is performed by conventional procedures with radiolabeled nucleotides or by automatic sequencing procedures with fluorescent tags.

Sequence changes at the specific locations may be revealed by nuclease protection assays, such as RNase and S1 protection or the chemical cleavage method (for example, Cotton et al., PNAS, 85:4397-4401 (1985)).

The receptor polypeptides of the present invention may also be employed in the process for screening for antagonists and/or agonists for the receptor. In general, such screening procedures involve providing appropriate cells which express the receptor on the surface thereof. In particular, a polynucleotide encoding the NGF/TNF receptor of the present invention is employed to transfect cells to thereby express the NGF/TNF receptor. Such transfection may be accomplished by procedures as hereinabove described.

In particular, a polynucleotide encoding a receptor polypeptide of the present invention is employed to transfect cells to thereby express the receptor polypeptide.

Thus, for example, such assay may be employed for screening for a receptor antagonist by contacting the cells which encode a polypeptide of the present invention. Inhibition of the signal generated by the ligand indicates that a compound is a potential antagonist for the receptor, i.e., inhibits activation of the receptor.

The screening may be employed for determining an agonist by contacting such cells with compounds to be screened and determining whether such compounds generate a signal, i.e., activates the receptor.

Other screening techniques include the use of cells which express the receptor polypeptides of the present invention (for example, transfected CHO cells) in a system which measures extracellular pH changes caused by receptor activation, for example, as described in Science, Volume 246, pages 181-296 (1989). In another example, potential agonists or antagonists may be contacted with a cell which expresses the receptor polypeptides of the present invention and a second messenger response, e.g., signal transduction may be measured to determine whether the potential antagonist or agonist is effective.

Another screening technique involves expressing the polypeptide of the present invention in which the polypeptide is linked to phospholipase C or D. As representative examples of such cells, there may be mentioned endothelial cells, smooth muscle cells, embryonic kidney cells and the like. The screening for an antagonist or agonist may be accomplished as hereinabove described by detecting activation of the receptor or inhibition of activation of the receptor from the phospholipase second signal

Antibodies may be utilized as both an agonist and an antagonist depending on the domain of the polypeptide of the present invention which the antibody binds to. The antibody in one instance can bind to the active site of the receptor and block ligand access. However, it has been observed that monoclonal antibodies directed against certain receptor polypeptides can act as specific agonists when binding to the extra-cellular domain of the receptor.

In addition to the antagonists identified above, oligonucleotides which bind to the receptor polypeptides of the present invention may also act as antagonists. Alternatively, a potential antagonist may be a soluble form of the polypeptides of the present invention which contains the complete extra-cellular region of the polypeptide and which binds to ligands to inhibit their biological activity.

Another potential antagonist to the receptor polypeptides of the present invention is an antisense construct prepared using antisense technology. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes for the mature polypeptides of the present invention, is used to design an antisense RNA oligonucleotide of from about 10 to 40 base pairs in length. A DNA oligonucleotide is designed to be complementary to a region of the gene involved in transcription (triple helix -see Lee et al., Nucl. Acids Res., 6:3073 (1979); Cooney et al, Science, 241:456 (1988); and Dervan et al., Science, 251: 1360 (1991)), thereby preventing transcription and the production of the polypeptides of the present invention. The antisense RNA oligonucleotide hybridizes to the mRNA *in vivo* and blocks translation of the mRNA molecule into the polypeptides of the present invention (antisense - Okano, J. Neurochem., 56:560 (1991) ; Oligodeoxynucleotides as Antisense Inhibitors of Gene Expression, CRC Press, Boca Raton, FL (1988)). The oligonucleotides described above can also be delivered to cells such that the antisense RNA or DNA may be expressed *in vivo* to inhibit production of the polypeptides of the present invention.

Potential antagonists to the polypeptides of the present invention also include a small molecule which binds to and occupies the receptor polypeptides thereby making them inaccessible to ligands which bind thereto such that normal biological activity is prevented. Examples of small molecules include but are not limited to small peptides or peptide-like molecules.

The agonists to the polypeptides of the present invention may be employed to stimulate ligand activities, such as inhibition of tumor growth and necrosis of certain transplantable tumors. The agonists may also be employed to stimulate cellular differentiation, for example, T-cell, fibroblasts and haemopoietic cell differentiation. Agonists to the receptor polypeptides may also augment TNF's role in the host's defense against microorganisms and prevent related diseases (infections such as that from L. monocytogenes) and chlamidiae. The agonists may also be employed to protect against the deleterious effects of ionizing radiation produced during a course of radiotherapy, such as denaturation of enzymes, lipid peroxidation, and DNA damage.

The agonists may also be employed to mediate an anti-viral response, to regulate growth, to mediate the immune response and to treat immunodeficiencies related to diseases such as HIV.

Antagonists to the receptor polypeptides of the present invention may be employed to treat autoimmune diseases, for example, graft versus host rejection and allograft rejection, and T-cell mediated autoimmune diseases such as AIDS. It has been shown that T-cell proliferation is stimulated via a type 2 TNF receptor. Accordingly, antagonizing the receptor may prevent the proliferation of T-cells and treat T-cell mediated autoimmune diseases.

The antagonists may also be employed to prevent apoptosis, which is the basis for diseases such as viral infection, rheumatoid arthritis, systemic lupus erythematosus, insulin-dependent diabetes mellitus, and graft rejection. Similarly, the antagonists may be employed to prevent cytotoxicity.

The antagonists to the receptor polypeptides of the present invention may also be employed to treat B cell cancers which are stimulated by TNF.

The soluble forms of the receptor polypeptides of the present invention may be employed to protect against arthritis, specifically arthritis induced by Type II collagen.

Antagonists to the receptor polypeptides of the present invention may also be employed to treat and/or prevent septic shock, which remains a critical clinical condition. Septic shock results from an exaggerated host response, mediated by protein factors such as TNF and IL-1, rather than from a pathogen directly. For example, lipopolysaccharides have been shown to elicit the release of TNF leading to a strong and transient increase of its serum concentration. TNF causes shock and tissue injury when administered in excessive amounts. Accordingly, antagonists to the receptor polypeptides of the present invention will block the actions of TNF and treat/prevent septic shock. These antagonists may also be used to treat meningococcemia in children which correlates with high serum levels of TNF.

Among other disorders which may be treated by the antagonists, there are included, inflammation which is mediated by ligands to the receptor polypeptides of the present invention, and the bacterial infections cachexia and cerebral malaria.

The soluble form of the receptor polypeptides of the present invention and agonists and antagonists may be employed in combination with a suitable pharmaceutical carrier. Such compositions comprise a therapeutically effective amount of the soluble receptor or agonist or antagonist, and a pharmaceutically acceptable carrier or excipient. Such a carrier includes but is not limited to saline, buffered saline, dextrose, water, glycerol, ethanol, and combinations thereof. The formulation should suit the mode of administration.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the soluble form of the receptor polypeptides of the present invention and agonists and antagonists of the present invention may also be employed in conjunction with other therapeutic compounds.

The pharmaceutical compositions may be administered in a convenient manner such as by the oral, topical, intravenous, intraperitoneal, intramuscular, subcutaneous, intranasal or intradermal routes. The pharmaceutical compositions are administered in an amount which is effective for treating and/or prophylaxis of the specific indication. In general, they are administered in an amount of at least about 10 µg/kg body weight and in most cases they will be administered in an amount not in excess of about 8 mg/Kg body weight per day. In most cases, the dosage is from about 10 µg/kg to about 1 mg/kg body weight daily, taking into account the routes of administration, symptoms, etc.

The receptor polypeptides of the present invention and agonists and antagonists which are polypeptides may also be employed in accordance with the present invention by expression of such polypeptides *in vivo*, which is often referred to as "gene therapy."

Thus, for example, cells from a patient may be engineered with a polynucleotide (DNA or RNA) encoding a polypeptide *ex vivo*, with the engineered cells then being provided to a patient to be treated with the polypeptide. Such methods are well-known in the art. For example, cells may be engineered by procedures known in the art by use of a retroviral particle containing RNA encoding a polypeptide of the present invention.

Similarly, cells may be engineered *in vivo* for expression of a polypeptide *in vivo* by, for example, procedures known in the art. As known in the art, a producer cell for producing a retroviral particle containing RNA encoding the polypeptide of the present invention may be administered to a patient for engineering cells *in vivo* and expression of the polypeptide *in vivo*. These and other methods for administering a polypeptide of the present invention by such method should be apparent to those skilled in the art from the teachings of the present invention. For example, the expression vehicle for engineering cells may be other than a retrovirus, for example, an adenovirus which may be used to engineer cells in vivo after combination with a suitable delivery vehicle.

The retroviral plasmid vectors may be derived from retroviruses which include, but are not limited to, Moloney Murine Sarcoma Virus, Moloney Murine Leukemia Virus, spleen necrosis virus, Rous Sarcoma Virus and Harvey Sarcoma Virus.

In a preferred embodiment the retroviral expression vector, pMV-7, is flanked by the long terminal repeats (LTRS) of the Moloney murine sarcoma virus and contains the selectable drug resistance gene neo under the regulation of the herpes simplex virus (HSV) thymidine kinase (tk) promoter. Univque EcoRI and HimdIII sites facilitate the introduction of coding sequence (Kirschmeier, P.T. et al., DNA, 7:219-25 (1988)).

The vectors include one or more suitable promoters which include, but are not limited to, the retroviral LTR; the SV40 promoter; and the human cytomegalovirus (CMV) promoter described in Miller, et al., Biotechniques, Vol. 7, No. 9, 980-990 (1989), or any other promoter (e.g., cellular promoters such as eukaryotic cellular promoters including, but not limited to, the histone, pol III, and β-actin promoters). The selection of a suitable promoter will be apparent to thsoe skilled in the art from the teachings contained herein.

The nucleic acid sequence encoding the polypeptide of the present invention is under the control of a suitable promoter which includes, but is not limited to, viral thymidine kinase promoters, such as the Herpes Simplex thymidine kinase promoter; retroviral LTRs, the β-actin promoter, and the native promoter which controls the gene encoding the polypeptide.

The retroviral plasmid vector is employed to transduce packaging cell lines to form producer cell lines. Examples of packaging cells which may be transfected include, but are not limited to, the PE501, PA317 and GP+am12. The vector may transduce the packaging cells through any means known in the art. Such means include, but are not limited to, electroporation, the use of liposomes, and CaPO₄ precipitation.

The producer cell line generates infectious retroviral vector particles which include the nucleic acid sequence(s) encoding the polypeptides. Such retroviral vector particles then may be employed, to transduce eukaryotic cells, either *in vitro* or *in vivo*. The transduced eukaryotic cells will express the nucleic acid sequence(s) encoding the polypeptide. Eukaryotic cells which may be transduced, include but are not limited to, fibroblasts and endothelial cells.

The sequences of the present invention are also valuable for chromosome identification. The sequence is specifically targeted to and can hybridize with a particular location on an individual human chromosome. Moreover, there is a current need for identifying particular sites on the chromosome. Few chromosome marking reagents based on actual sequence data (repeat polymorphisms) are presently available for marking chromosomal location. The mapping of DNAs to chromosomes according to the present invention is an important first step in correlating those sequences with genes associated with disease.

Briefly, sequences can be mapped to chromosomes by preparing PCR primers (preferably 15-25 bp) from the cDNA. Computer analysis of the 3' untranslated region is used to rapidly select primers that do not span more than one exon in the genomic DNA, thus complicating the amplification process. These primers are then used for PCR screening of somatic cell hybrids containing individual human chromosomes. Only those hybrids containing the human gene corresponding to the primer will yield an amplified fragment.

PCR mapping of somatic cell hybrids is a rapid procedure for assigning a particular DNA to a particular chromosome. Using the present invention with the same oligonucleotide primers, sublocalization can be achieved with panels of fragments from specific chromosomes or pools of large genomic clones in an analogous manner. Other mapping strategies that can similarly be used to map to its chromosome include in situ hybridization, prescreening with labeled flow-sorted chromosomes and preselection by hybridization to construct chromosome specific-cDNA libraries.

Fluorescence in situ hybridization (FISH) of a cDNA clone to a metaphase chromosomal spread can be used to provide a precise chromosomal location in one step. This technique can be used with cDNA having at least 500 or 600 bases. For a review of this technique, see Verma et al., Human Chromosomes: a Manual of Basic Techniques, Pergamon Press, New York (1988).

Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes).

Next, it is necessary to determine the differences in the cDNA or genomic sequence between affected and unaffected individuals. If a mutation is observed in some or all of the affected individuals but not in any normal individuals, then the mutation is likely to be the causative agent of the disease.

With current resolution of physical mapping and genetic mapping techniques, a cDNA precisely localized to a chromosomal region associated with the disease could be one of between 50 and 500 potential causative genes. (This assumes 1 megabase mapping resolution and one gene per 20 kb) .

The polypeptides, their fragments or other derivatives, or analogs thereof, or cells expressing them can be used as an immunogen to produce antibodies thereto. These antibodies can be, for example, polyclonal or monoclonal antibodies. The present invention also includes chimeric, single chain, and humanized antibodies, as well as Fab fragments, or the product of an Fab expression library. Various procedures known in the art may be used for the production of such antibodies and fragments.

Antibodies generated against the polypeptides corresponding to a sequence of the present invention can be obtained by direct injection of the polypeptides into an animal or by administering the polypeptides to an animal, preferably a nonhuman. The antibody so obtained will then bind the polypeptides itself. In this manner, even a sequence encoding only a fragment of the polypeptides can be used to generate antibodies binding the whole native polypeptides. Such antibodies can then be used to isolate the polypeptide from tissue expressing that polypeptide.

For preparation of monoclonal antibodies, any technique which provides antibodies produced by continuous cell line cultures can be used. Examples include the hybridoma technique (Kohler and Milstein, 1975, Nature, 256:495-497), the trioma technique, the human B-cell hybridoma technique (Kozbor et al., 1983, Immunology Today 4:72), and the EBV-hybridoma technique to produce human monoclonal antibodies (Cole, et al., 1985, in Monoclonal Antibodies and Cancer Therapy, Alan R. Liss, Inc., pp. 77-96).

Techniques described for the production of single chain antibodies (U.S. Patent 4,946,778) can be adapted to produce single chain antibodies to immunogenic polypeptide products of this invention. Also, transgenic mice may be used to express humanized antibodies to immunogenic polypeptide products of this invention.

The present invention will be further described with reference to the following examples; however, it is to be understood that the present invention is not limited to such examples. All parts or amounts, unless otherwise specified, are by weight.

In order to facilitate understanding of the following examples certain frequently occurring methods and/or terms will be described.

"Plasmids" are designated by a lower case p preceded and/or followed by capital letters and/or numbers. The starting plasmids herein are either commercially available, publicly available on an unrestricted basis, or can be constructed from available plasmids in accord with published procedures. In addition, equivalent plasmids to those described are known in the art and will be apparent to the ordinarily skilled artisan.

"Digestion" of DNA refers to catalytic cleavage of the DNA with a restriction enzyme that acts only at certain sequences in the DNA. The various restriction enzymes used herein are commercially available and their reaction conditions, cofactors and other requirements were used as would be known to the ordinarily skilled artisan. For analytical purposes, typically 1 µg of plasmid or DNA fragment is used with about 2 units of enzyme in about 20 µl of buffer solution. For the purpose of isolating DNA fragments for plasmid construction, typically 5 to 50 µg of DNA are digested with 20 to 250 units of enzyme in a larger volume. Appropriate buffers and substrate amounts for particular restriction enzymes are specified by the manufacturer. Incubation times of about 1 hour at 37 ° C are ordinarily used, but may vary in accordance with the supplier's instructions. After digestion the reaction is electrophoresed directly on a polyacrylamide gel to isolate the desired fragment.

Size separation of the cleaved fragments is performed using 8 percent polyacrylamide gel described by Goeddel, D. et al., Nucleic Acids Res., 8:4057 (1980).

"Oligonucleotides" refers to either a single stranded polydeoxynucleotide or two complementary polydeoxynucleotide strands which may be chemically synthesized. Such synthetic oligonucleotides have no 5' phosphate and thus will not ligate to another oligonucleotide without adding a phosphate with an ATP in the presence of a kinase. A synthetic oligonucleotide will ligate to a fragment that has not been dephosphorylated.

"Ligation" refers to the process of forming phosphodiester bonds between two double stranded nucleic acid fragments (Maniatis, T., et al., Id., p. 146). Unless otherwise provided, ligation may be accomplished using known buffers and conditions with 10 units of T4 DNA ligase ("ligase") per 0.5 µg of approximately equimolar amounts of the DNA fragments to be ligated.

Unless otherwise stated, transformation was performed as described in the method of Graham, F. and Van der Eb, A., Virology, 52:456-457 (1973).

### Example 1

### Bacterial Expression and Purification of the TNF receptor

The DNA sequence encoding TNF receptor, ATCC # 97059, is initially amplified using PCR oligonucleotide primers corresponding to the 5' and 3' end sequences of the processed TNF receptor nucleic acid sequence (minus the signal peptide sequence). Additional nucleotides corresponding to TNF receptor gene are added to the 5' and 3' end sequences respectively. The 5' oligonucleotide primer has the sequence 5' GCGCGGATCCGAGCCTCCTGGAGACTGG 3' (SEQ ID No. 7) contains a BamHI restriction enzyme site (bold) followed by 18 nucleotides of TNF receptor coding sequence starting from the presumed initiation codon. The 3' sequence 5' GCGCAAGCTTTCAGTGGTTTGGGCTCCTCCC 3' (SEQ ID No. 8) contains complementary sequences to a Hind III site (bold) and is followed by 18 nucleotides of TNF receptor. The restriction enzyme sites correspond to the restriction enzyme sites on the bacterial expression vector pQE-9 (Qiagen, Inc. Chatsworth, CA). pQE-9 encodes antibiotic resistance (Amp'), a bacterial origin of replication (ori), an IPTG-regulatable promoter operator (P/O), a ribosome binding site (RBS), a 6-His tag and restriction enzyme sites. pQE-9 is then digested with BamHI and Hind III. The amplified sequences are ligated into pQE-9 and are inserted in frame with the sequence encoding for the histidine tag and the RES. The ligation mixture is then used to transform E. coli strain M15/rep 4 (Qiagen, Inc.) by the procedure described in Sambrook, J. et al., Molecular Cloning: A Laboratory Manual, Cold Spring Laboratory Press, (1989). M15/rep4 contains multiple copies of the plasmid pREP4, which expresses the lacI repressor and also confers kanamycin resistance (Kan'). Transformants are identified by their ability to grow on LB plates and ampicillin/kanamycin resistant colonies are selected. Plasmid DNA is isolated and confirmed by restriction analysis. Clones containing the desired constructs are grown overnight (O/N) in liquid culture in LB media supplemented with both Amp (100 ug/ml) and Kan (25 ug/ml). The O/N culture is used to inoculate a large culture at a ratio of 1:100 to 1:250. The cells are grown to an optical density 600 (O.D.⁶⁰⁰) of between 0.4 and 0.6. IPTG ("Isopropyl-B-D-thiogalacto pyranoside") is then added to a final concentration of 1 mM. IPTG induces by inactivating the lacI repressor, clearing the P/O leading to increased gene expression. Cells are grown an extra 3 to 4 hours. Cells are then harvested by centrifugation. The cell pellet is solubilized in the chaotropic agent 6 Molar Guanidine HCl. After clarification, solubilized TNF receptor is purified from this solution by chromatography on a Nickel-Chelate column under conditions that allow for tight binding by proteins containing the 6-His tag (Hochuli, E. et al., J. Chromatography 411:177-184 (1984)). TNF receptor is eluted from the column in 6 molar guanidine HCl pH 5.0 and for the purpose of renaturation adjusted to 3 molar guanidine HCl, 100mM sodium phosphate, 10 mmolar glutathione (reduced) and 2 mmolar glutathione (oxidized). After incubation in this solution for 12 hours the protein is dialyzed to 10 mmolar sodium phosphate.

### Example 2

### Cloning and expression of the TNF receptor and extracellular (soluble) TNF soluble form of the TNF receptor using the baculovirus expression system

The DNA sequence encoding the full length TNF receptor protein, ATCC # 97059, and the soluble receptor nucleic acid are amplified using PCR oligonucleotide primers corresponding to the 5' and 3' sequences of the gene (throughout this example TNF receptor is meant to include both the full-length receptor and the soluble form of the receptor) :

The 5' primer has the sequence 5' GCGCGGATCCACCATGGAGCCTCCTGGAGACTGG 3' (SEQ ID No. 9) and contains a BamHI restriction enzyme site (in bold) followed by 3 nucleotides resembling an efficient signal for the initiation of translation in eukaryotic cells (Kozak, M., J. Mol. Biol., 196:947-950 (1987) and which is just behind the first 21 nucleotides of the TNF receptor gene (the initiation codon for translation "ATG" is underlined).

The 3' primer has the sequence 5' GCGCGGTACCTCAGTGG GAGCTGCTGGTCCC 3' (SEQ ID No. 10) (for extracellular domain) and 5' GCGCGGTACCTCAGTGGTTTGGGCTCCTCCC 3' (SEQ ID No. 11) (for full-length receptor) and contains the cleavage site for the restriction endonuclease Asp 718 and 21 nucleotides complementary to the 3' non-translated sequence of the TNF receptor gene. The amplified sequences are isolated from a 1% agarose gel using a commercially available kit ("Geneclean", BIO 101 Inc., La Jolla, Ca.). The fragments are then digested with the endonucleases BamHI and Asp 718 and then purified again on a 1% agarose gel. This fragment is designated F2.

The vector pRG1 (modification of pVL941 vector, discussed below) is used for the expression of the TNF receptor proteins using the baculovirus expression system (for review see: Summers, M.D. and Smith, G.E. 1987, A manual of methods for baculovirus vectors and insect cell culture procedures, Texas Agricultural Experimental Station Bulletin No. 1555). This expression vector contains the strong polyhedrin promoter of the Autographa californica nuclear polyhedrosis virus (AcMNPV) followed by the recognition sites for the restriction endonucleases BamHI and Asp 718. The polyadenylation site of the simian virus (SV)40 is used for efficient polyadenylation. For an easy selection of recombinant viruses the beta-galactosidase gene from E.coli is inserted in the same orientation as the polyhedrin promoter followed by the polyadenylation signal of the polyhedrin gene. The polyhedrin sequences are flanked at both sides by viral sequences for the cell-mediated homologous recombination of cotransfected wild-type viral DNA. Many other baculovirus vectors could be used in place of pRG1 such as pAc373, pVL941 and pAcIM1 (Luckow, V.A. and Summers, M.D., Virology, 170:31-39).

The plasmid is digested with the restriction enzymes BamHI and Asp 718 and then dephosphorylated using calf intestinal phosphatase by procedures known in the art. The DNA is then isolated from a 1% agarose gel using the commercially available kit ("Geneclean" BIO 101 Inc., La Jolla, Ca.). This vector DNA is designated V2.

Fragment F2 and the dephosphorylated plasmid V2 are ligated with T4 DNA ligase. Sf9 cells are then transformed and cells identified that contained the plasmid (pBac TNF receptor) with the TNF receptor genes using the enzymes BamHI and Asp 718. The sequence of the cloned fragment is confirmed by DNA sequencing.

5 µg of the plasmid pBac TNF receptor is cotransfected with 1.0 µg of a commercially available linearized baculovirus ("BaculoGold™ baculovirus DNA", Pharmingen, San Diego, CA.) using the lipofection method (Felgner et al. Proc. Natl. Acad. Sci. USA, 84:7413-7417 (1987)).

1µg of BaculoGold™ virus DNA and 5 µg of the plasmid pBac TNF receptors are mixed in a sterile well of a microtiter plate containing 50 µl of serum free Grace's medium (Life Technologies Inc., Gaithersburg, MD). Afterwards 10 µl Lipofectin plus 90 µl Grace's medium are added, mixed and incubated for 15 minutes at room temperature. Then the transfection mixture is added dropwise to the Sf9 insect cells (ATCC CRL 1711) seeded in a 35 mm tissue culture plate with 1 ml Grace's medium without serum. The plate is rocked back and forth to mix the newly added solution. The plate is then incubated for 5 hours at 27°C. After 5 hours the transfection solution is removed from the plate and 1 ml of Grace's insect medium supplemented with 10% fetal calf serum is added. The plate is put back into an incubator and cultivation continued at 27°C for four days.

After four days the supernatant is collected and a plaque assay performed similar as described by Summers and Smith (supra). As a modification an agarose gel with "Blue Gal" (Life Technologies Inc., Gaithersburg) is used which allows an easy isolation of blue stained plaques. (A detailed description of a "plaque assay" can also be found in the user's guide for insect cell culture and baculovirology distributed by Life Technologies Inc., Gaithersburg, page 9-10) .

Four days after the serial dilution, the viruses are added to the cells and blue stained plaques are picked with the tip of an Eppendorf pipette. The agar containing the recombinant viruses are then resuspended in an Eppendorf tube containing 200 µl of Grace's medium. The agar is removed by a brief centrifugation and the supernatant containing the recombinant baculoviruses is used to infect Sf9 cells seeded in 35 mm dishes. Four days later the supernatants of these culture dishes are harvested and then stored at 4°C.

Sf9 cells are grown in Grace's medium supplemented with 10% heat-inactivated FBS. The cells are infected with the recombinant baculovirus V-TNF receptors at a multiplicity of infection (MOI) of 2. Six hours later the medium is removed and replaced with SF900 II medium minus methionine and cysteine (Life Technologies Inc., Gaithersburg). 42 hours later 5 µCi of ³⁵S-methionine and 5 µCi ³⁵S cysteine (Amersham) are added. The cells are further incubated for 16 hours before they are harvested by centrifugation and the labelled proteins visualized by SDS-PAGE and autoradiography.

### Example 3

### Expression of Recombinant TNF receptor in COS cells

The expression of plasmid, TNF receptor HA is derived from a vector pcDNAI/Amp (Invitrogen) containing: 1) SV40 origin of replication, 2) ampicillin resistance gene, 3) E.coli replication origin, 4) CMV promoter followed by a polylinker region, a SV40 intron and polyadenylation site. A DNA fragment encoding the entire TNF receptor precursor and a HA tag fused in frame to its 3' end is cloned into the polylinker region of the vector, therefore, the recombinant protein expression is directed under the CMV promoter. The HA tag correspond to an epitope derived from the influenza hemagglutinin protein as previously described (I. Wilson, H. Niman, R. Heighten, A Cherenson, M. Connolly, and R. Lerner, 1984, Cell 37, 767). The infusion of HA tag to the target protein allows easy detection of the recombinant protein with an antibody that recognizes the HA epitope.

The plasmid construction strategy is described as follows:

The DNA sequence encoding TNF receptor, ATCC # 97059, is constructed by PCR using two primers: the 5' primer 5' GCGCGGATCCACCATGGAGCCTCCTGGAGACTGG 3' (SEQ ID No. 12) contains a BamHI site (bold) followed by 21 nucleotides of TNF receptor coding sequence starting from the initiation codon; the 3' sequence 5' GCGCTCTAGATCAAGCGTAGTCTGG GACGTCGTATGGGTAGTGGTTTGGGCTCCTCCC 3'(SEQ ID No. 13) contains complementary sequences to an XbaI site (bold), translation stop codon, HA tag and the last 18 nucleotides of the TNF receptor coding sequence (not including the stop codon). Therefore, the PCR product contains a BamHI site, TNF receptor coding sequence followed by HA tag fused in frame, a translation termination stop codon next to the HA tag, and an XbaI site. The PCR amplified DNA fragment and the vector, pcDNAI/Amp, are digested with BamHI and XbaI restriction enzyme and ligated. The ligation mixture is transformed into E. coli strain SURE (Stratagene Cloning Systems, La Jolla, CA) the transformed culture is plated on ampicillin media plates and resistant colonies are selected. Plasmid DNA is isolated from transformants and examined by restriction analysis for the presence of the correct fragment. For expression of the recombinant TNF receptor, COS cells are transfected with the expression vector by DEAE-DEXTRAN method (J. Sambrook,.E. Fritsch, T. Maniatis, Molecular Cloning: A Laboratory Manual, Cold Spring Laboratory Press, (1989)). The expression of the TNF receptor HA protein is detected by radiolabelling and immunoprecipitation method (E. Harlow, D. Lane, Antibodies: A Laboratory Manual, Cold Spring Harbor Laboratory Press, (1988)). Cells are labelled for 8 hours with ³⁵S-cysteine two days post transfection. Culture media are then collected and cells are lysed with detergent (RIPA buffer (150 mM NaCl, 1% NP-40, 0.1% SDS, 1% NP-40, 0.5% DOC, 50mM Tris, pH 7.5) (Wilson, I. et al. , Id. 37:767 (1984)) . Both cell lysate and culture media are precipitated with a HA specific monoclonal antibody. Proteins precipitated are analyzed on 15% SDS-PAGE gels.

### Example 4

### Expression pattern of TNF receptor in human tissue

Northern blot analysis is carried out to examine the levels of expression of TNF receptor in human tissues. Total cellular RNA samples are isolated with RNAzoI™ B system (Biotecx Laboratories, Inc. Houston, TX). About 10µg of total RNA isolated from each human tissue specified is separated on 1% agarose gel and blotted onto a nylon filter. (Sambrook, Fritsch, and Maniatis, Molecular Cloning, Cold Spring Harbor Press, (1989)). The labeling reaction is done according to the Stratagene Prime-It kit with 50ng DNA fragment. The labeled DNA is purified with a Select-G-50 column. (5 Prime - 3 Prime, Inc. Boulder, CO). The filter is then hybridized with radioactive labeled full length TNF receptor gene at 1,000,000 cpm/ml in 0.5 M NaPO₄, pH 7.4 and 7% SDS overnight at 65°C. After wash twice at room temperature and twice at 60°C with 0.5 x SSC, 0.1% SDS, the filter is then exposed at -70°C overnight with an intensifying screen.

### Example 5

### Expression of TNF Receptor via Gene Therapy

Fibroblasts are obtained from a subject by skin biopsy. The resulting tissue is placed in tissue-culture medium and separated into small pieces. Small chunks of the tissue are placed on a wet surface of a tissue culture flask, approximately ten pieces are placed in each flask. The flask is turned upside down, closed tight and left at room temperature over night. After 24 hours at room temperature, the flask is inverted and the chunks of tissue remain fixed to the bottom of the flask and fresh media (e.g., Ham's F12 media, with 10% FBS, penicillin and streptomycin, is added. This is then incubated at 37°C for approximately one week. At this time, fresh media is added and subsequently changed every several days. After an additional two weeks in culture, a monolayer of fibroblasts emerge. The monolayer is trypsinized and scaled into larger flasks.

pMV-7 (Kirschmeier, P.T. et al, DNA, 7:219-25 (1988) flanked by the long terminal repeats of the Moloney murine sarcoma virus, is digested with EcoRI and HindIII and subsequently treated with calf intestinal phosphatase. The linear vector is fractionated on agarose gel and purified, using glass beads.

The TNF Recpetor cDNA is amplified using PCR primers which correspond to the 5' and 3' end sequences respectively. The 5' primer containing an EcoRI site and the 3' primer having contains a HindIII site. Equal quantities of the Moloney murine sarcoma virus linear backbone and the EcoRI and HimdIII fragment are added together, in the presence of T4 DNA ligase. The resulting mixture is maintained under conditions appropriate for ligation of the two fragments. The ligation mixture is used to transform bacteria HB101, which are then plated onto agar-containing kanamycin for the purpose of confirming that the vector had the TNF Receptor gene properly inserted.

The amphotropic pA317 or GP+am12 packaging cells are grown in tissue culture to confluent density in Dulbecco's Modified Eagles Medium (DMEM) with 10% calf serum (CS), penicillin and streptomycin. The MSV vector containing the TNF Receptor gene is then added to the media and the packaging cells are transduced with the vector. The packaging cells now produce infectious viral particles containing the TNF Receptor gene (the packaging cells are now referred to as producer cells).

Fresh media is added to the transduced producer cells, and subsequently, the media is harvested from a 10 cm plate of confluent producer cells. The spent media, containing the infectious viral particles, is filtered through a millipore filter to remove detached producer cells and this media is then used to infect fibroblast cells. Media is removed from a sub-confluent plate of fibroblasts and quickly replaced with the media from the producer cells. This media is removed and replaced with fresh media. If the titer of virus is high, then virtually all fibroblasts will be infected and no selection is required. If the titer is very low, then it is necessary to use a retroviral vector that has a selectable marker, such as neo or his.

The engineered fibroblasts are then injected into the host, either alone or after having been grown to confluence on cytodex 3 microcarrier beads. The fibroblasts now produce the TNF Receptor protein product.

The DNA sequence encoding for TNF-SV1 and TNF-SV2, ATCC Nos. 97058 and 97057, may also be expressed in the above-described assays by adjusting the primer sequences which is within the knowledge of a person of ordinary skill in the art.

Numerous modifications and variations of the present invention are possible in light of the above teachings and, therefore, within the scope of the appended claims, the invention may be practiced otherwise than as particularly described.

WHAT IS ITEMIZED IS :
1. An isolated polynucleotide comprising a member selected from the group consisting of:
   (a) a polynucleotide encoding the polypeptide comprising amino acid -38 to amino acid 245 as set forth in SEQ ID No. 2;
   (b) a polynucleotide encoding the polypeptide comprising amino acid 1 to amino acid 245 as set forth in SEQ ID No. 2;
   (c) a polynucleotide encoding the polypeptide comprising amino acid -38 to amino acid 202 as set forth in SEQ ID No. 4;
   (d) a polynucleotide comprising amino acid 1 to amino acid 202 as set forth in SEQ ID No. 4;
   (e) a polynucleotide comprising amino acid 1 to amino acid 134 as set forth in SEQ ID No. 6;
   (f) a polynucleotide capable of hybridizing to and which is at least 70% identical to the polynucleotide of (a), (b), (c), (d), (e); and
   (g) a polynucleotide fragment of the polynucleotide of (a), (b), (c), (d), (e) and (f).
2. The polynucleotide of Item 1 encoding the polypeptide comprising amino acid -38 to amino acid 245 as set forth in SEQ ID NO. 2.
3. The polynucleotide of Item 1 encoding the polypeptide comprising amino acid 1 to amino acid 245 as set forth in SEQ ID No. 2.
4. The polynucleotide of Item 1 encoding the polypeptide comprising amino acid -38 to amino acid 202 as set forth in SEQ ID NO. 4.
5. The polynucleotide of Item 1 encoding the polypeptide comprising amino acid 1 to amino acid 202 as set forth in SEQ ID No. 4.
6. The polynucleotide of Item 1 encoding the polypeptide comprising amino acid 1 to amino acid 134 as set forth in SEQ ID No. 6.
7. The polynucleotide of Item 1 wherein the polynucleotide is DNA.
8. The polynucleotide of Item 1 wherein said polynucleotide is RNA.
9. The polynucleotide of Item 1 wherein the polynucleotide is genomic DNA.
10. The polynucleotide of Item 1 comprising from nucleotide 1 to nucleotide 881 as set forth in SEQ ID NO. 1.
11. The polynucleotide of Item 1 comprising from nucleotide 123 to nucleotide 855 as set forth in SEQ ID NO. 1.
12. The polynucleotide of Item 1 comprising from nucleotide 1 to nucleotide 723 as set forth in SEQ ID NO. 3.
13. The polynucleotide of Item 1 comprising from nucleotide 115 to nucleotide 723 as set forth in SEQ ID NO. 3.
14. The polynucleotide of Item 1 comprising from nucleotide 1 to nucleotide 405 as set forth in SEQ ID NO. 5.
15. An isolated polynucleotide comprising a member selected from the group consisting of:
   (a) a polynucleotide encoding a mature polypeptide encoded by the DNA contained in ATCC Deposit No. 97059;
   (b) a polynucleotide encoding the polypeptide expressed by the DNA contained in ATCC Deposit No. 97059;
   (c) a polynucleotide encoding a mature polypeptide encoded by the DNA contained in ATCC Deposit No. 97058;
   (d) a polynucleotide encoding the polypeptide expressed by the DNA contained in ATCC Deposit No. 97058;
   (e) a polynucleotide encoding a mature polypeptide encoded by the DNA contained in ATCC Deposit No. 97057;
   (f) a polynucleotide encoding the polypeptide expressed by the DNA contained in ATCC Deposit No. 97057;
   (g) a polynucleotide capable of hybridizing to and which is at least 70% identical to the polynucleotide of (a), (b) , (c) , (d) , (e) , or (f) ; and
   (h) a polynucleotide fragment of the polynucleotide of (a), (b) , (c), (d), (e), (f) or (g).
16. The polynucleotide of Item 15 wherein said polynucleotide encodes a polypeptide expressed by the DNA contained in ATCC Deposit No. 97059.
17. The polynucleotide of Item 15 wherein said polynucleotide encodes a polypeptide expressed by the DNA contained in ATCC Deposit No. 97058.
18. The polynucleotide of Item 15 wherein said polynucleotide encodes a polypeptide expressed by the DNA contained in ATCC Deposit No. 97057.
19. A vector containing the DNA of Item 2.
20. A host cell genetically engineered with the vector of Item 19.
21. A process for producing a polypeptide comprising: expressing from the host cell of Item 20 the polypeptide encoded by said DNA.
22. A process for producing cells capable of expressing a polypeptide comprising genetically engineering cells with the vector of Item 19.
23. A polypeptide selected from the group consisting of (i) a polypeptide having the deduced amino acid sequence of SEQ ID No. 2 and fragments, analogs and derivatives thereof; (ii) a polypeptide encoded by the cDNA of ATCC Deposit No. 97059 and fragments, analogs and derivatives of said polypeptide; (iii) a polypeptide having the deduced amino acid sequence of SEQ ID No. 4 and fragments, analogs and derivatives thereof; (iv) a polypeptide encoded by the cDNA of ATCC Deposit No. 97058 and fragments, analogs and derivatives of said polypeptide; (v) a polypeptide having the deduced amino acid sequence of SEQ ID No. 6 and fragments, analogs and derivatives thereof; and (vi) a polypeptide encoded by the cDNA of ATCC Deposit No. 97057 and fragments, analogs and derivatives of said polypeptide.
24. The polypeptide of Item 23 wherein the polypeptide has the deduced amino acid sequence of SEQ ID No. 2.
25. The polypeptide of Item 23 wherein the polypeptide has the deduced amino acid sequence of SEQ ID No. 4.
26. The polypeptide of Item 23 wherein the polypeptide has the deduced amino acid sequence of SEQ ID No. 6.
27. An antibody against the polypeptide of Item 23.
28. A compound which activates the polypeptide of claim 23.
29. A compound which inhibits activation the polypeptide of Item 23.
30. A method for the treatment of a patient having need to activate a TNF receptor comprising: administering to the patient a therapeutically effective amount of the compound of Item 28.
31. A method for the treatment of a patient having need to inhibit a TNF receptor comprising: administering to the patient a therapeutically effective amount of the compound of Item 29 .
32. The method of Item 30 wherein said compound is a polypeptide and a therapeutically effective amount of the compound is administered by providing to the patient DNA encoding said agonist and expressing said agonist *in vivo*.
33. The method of Item 31 wherein said compound is a polypeptide and a therapeutically effective amount of the compound is administered by providing to the patient DNA encoding said antagonist and expressing said antagonist *in vivo*.
34. A method for identifying agonists and antagonists to the polypeptide of Item 23 comprising: combining the polypeptide, a ligand known to bind to the polypeptide, and a compound to be screened under conditions where the ligand binds to the polypeptide and determining whether the compound inhibits or enhances a signal generated by the polypeptide.
35. A process for diagnosing a disease or a susceptibility to a disease related to an under-expression of the polypeptide of Item 23 comprising:
   determining a mutation in the nucleic acid sequence encoding said polypeptide.
36. The polypeptide of Item 23 wherein the polypeptide is a soluble fragment of the polypeptide and is capable of binding a ligand for the receptor.
37. A diagnostic process comprising:
   analyzing for the presence of the polypeptide of Item 36 in a sample derived from a host.
38. A process for determining whether a ligand not known to be capable of binding to the polypeptide of Item 23 can bind thereto comprising:
   contacting a mammalian cell which expresses the polypeptide with a potential ligand;
   detecting the presence of the ligand which binds to the polypeptide; and
   determining whether the ligand binds to the polypeptide.

## Claims

1. An antibody against a polypeptide having the amino acid sequence encoded by a polynucleotide selected from the group consisting of
(a) polynucleotides encoding at least the mature form or extracellular region of the polypeptide having the deduced amino acid sequence as shown in SEQ ID No. 2;
(b) polynucleotides having the coding sequence as shown in SEQ ID No. 1 encoding at least the mature form or extracellular region of the polypeptide;
(c) polynucleotides encoding the polypeptide having the amino acid sequence of at least the mature form or extracellular region of the polypeptide encoded by the cDNA contained in ATCC 97059;
(d) polynucleotides having the coding sequence of the cDNA contained in ATCC 97059 encoding at least the mature form or extracellular region of the polypeptide; and
(e) polynucleotides the complementary strand of which hybridizes with and is at least 70% identical to a polynucleotide as defined in any one of (a) to (d) and which encode a polypeptide having Tumor Necrosis Factor (TNF) receptor activity or the extracellular region or a soluble fragment thereof capable of binding a ligand for the receptor;
or obtainable by a process for producing a polypeptide having TNF receptor activity or a soluble fragment thereof comprising culturing a host cell genetically engineered with a vector containing the polynucleotide as defined in any one of (a) to (e) and recovering the polypeptide encoded by said polynucleotide from the culture.

2. A pharmaceutical composition comprising an antibody of claim 1 or a DNA encoding and capable of expressing said antibody in vivo and optionally a pharmaceutically acceptable carrier.

3. A diagnostic composition comprising the antibody of claim 1.

4. A pharmaceutical composition comprising an antibody of claim 1 for the inhibition of tumor growth, for the stimulation of human cellular proliferation, e.g., T-cell proliferation, for regulating the immune response or antiviral responses, for the protection against the effects of ionizing radiation or chlamidiae infection, for the regulation of growth or for the treatment of immunodeficiencies.

5. A pharmaceutical composition comprising an antibody of claim 1 for treating T-cell mediated autoimmune diseases such as AIDS and arthritis, septic shock, cerebral malaria, graft rejection, cytotoxicity, cachexia, apoptosis or inflammation.
